# EUROPÄISCHE PATENTSCHRIFT

(11) **EP 3 407 772 B1**
(45) Veröffentlichungstag und Bekanntmachung des Hinweises auf die Patenterteilung: **09.06.2021**
(21) Anmeldenummer: 17706143.9
(22) Anmeldetag: 25.01.2017
(51) Int. Cl.: A61B 1/00, A61B 1/06, A61B 1/07, G02B 23/24, G02B 6/26, G02B 6/42

(54) **BELEUCHTUNGSEINRICHTUNG**
ILLUMINATING DEVICE
APPAREIL D'ÉCLAIRAGE

(30) Priorität: 29.01.2016 DE 102016201324
(43) Veröffentlichungstag der Anmeldung: 05.12.2018
(73) Patentinhaber: Richard Wolf GmbH, 75438 Knittlingen (DE)
(72) Erfinder: WEBER, Bernd Claus, 76228 Karlsruhe (DE); TEWES, Moritz, 75015 Bretten (DE); HOFFMANN, Marco, 68753 Waghäusel (DE); GÖTZ, Dieter, 75015 Bretten (DE)
(74) Vertreter: Patentanwälte Vollmann Hemmer Lindfeld Partnerschaft mbB
(86) Internationale Anmeldenummer: PCT/DE2017/200007
(87) Internationale Veröffentlichungsnummer: WO 2017/129186

(56) Entgegenhaltungen:
- EP-A1- 0 083 527
- EP-A1- 0 224 282
- EP-A2- 1 491 815
- DE-A1-102005 018 336
- GB-A- 2 169 096
- US-A- 3 779 628
- US-A- 3 832 028
- US-A1- 2005 046 807
- US-A1- 2010 176 311

## Beschreibung

Die Erfindung betrifft eine Beleuchtungseinrichtung mit den im Oberbegriff von Anspruch 1 angegebenen Merkmalen.

Den Ausgangspunkt der Erfindung bilden solche Beleuchtungseinrichtungen, bei denen eine LED in Verbindung mit einem Lichtleiter eingesetzt wird, um das von der LED emittierte Licht zu einem von der LED entfernten Ort zu transportieren. Derartigen Beleuchtungseinrichtungen kommt heutzutage auf unterschiedlichen technischen Gebieten eine große Bedeutung zu. Beispielhaft seien in diesem Zusammenhang Endoskope genannt, bei denen zu Beleuchtungszwecken Licht an dem distalen Ende eines länglichen Schaftes bereitgestellt werden muss, wobei die LED entweder als Teil des Endoskops oder als Teil einer separaten, mit dem Endoskop verbindbaren Lichtquelle proximalseitig des Schaftes angeordnet ist.

Ist an dem von der LED abgewandten Ende eine große Lichtausbeute erforderlich, müssen bereits von der LED entsprechend große Lichtströme bereitgestellt werden und in den Lichtleiter eingekoppelt werden. Dies führt an dem Ort der Lichteinkopplung in den Lichtleiter zu einer vergleichsweise hohen thermischen Belastung, welche darauf zurückzuführen ist, dass auch solche von der LED emittierte Lichtstrahlen den Lichtleiter erreichen, die einen vergleichsweise großen Winkel zu der optischen Achse des Lichtleiters bzw. zu der optischen Achse deren Fasern aufweisen und aufgrund der verhältnismäßig kleinen numerischen Apertur des Lichtleiters bzw. dessen Fasern nicht durch den Lichtleiter übertragen werden können und stattdessen absorbiert und in Wärme umgewandelt werden. Diese Wärmebelastung kann ungünstigstenfalls zu einer Beschädigung des Lichtleiters führen.

Die Erfindung geht aus von einem Stand der Technik wie er aus EP 0 083 527 A1 bekannt ist. Dort erfolgt die Einkopplung des von der LED emittierten Lichts in einen Lichtleiter mittels eines Lichtübertragungsteils, welches zwei Abschnitte aufweist, von denen ein erster Abschnitt direkt der LED zugewandt ist und einen sich mit zunehmendem Abstand von der LED erweiterten Querschnitt sowie eine die in den ersten Abschnitt eintretende Strahlung weitestgehend winkelunabhängig reflektierende Mantelfläche aufweist und von denen ein zweiter Abschnitt direkt dem Lichtleiter zugewandt ist, der einen in Richtung der Mittelachse des Lichtübertragungsteils konstanten Querschnitt aufweist und der zumindest eine die von dem ersten Abschnitt in den zweiten Abschnitt eintretende Strahlung weitestgehend winkelabhängig reflektierende Mantelfläche aufweist. Dabei ist der Ausgangsdurchmesser des zweiten Abschnitts deutlich größer als der Durchmesser des Lichtleiters.

Vor diesem Hintergrund liegt der Erfindung die Aufgabe zugrunde, eine Beleuchtungseinrichtung der vorab beschriebenen Art zu schaffen, welche ausgangsseitig des Lichtleiters hohe Lichtströme bereitstellt, wobei gleichzeitig die thermische Belastung des Lichtleiters in dem Bereich der Einkopplung des von der LED emittierten Lichts in den Lichtleiter möglichst gering ist.

Diese Aufgabe wird durch eine Beleuchtungseinrichtung mit den in Anspruch 1 angegebenen Merkmalen gelöst, wobei sich vorteilhafte Weiterbildungen dieser Beleuchtungseinrichtung aus den Unteransprüchen, der nachfolgenden Beschreibung sowie der Zeichnung ergeben. Hierbei können die in den Unteransprüchen angegebenen Merkmale vorteilhaft in der angegebenen Kombination aber auch, soweit technisch sinnvoll, für sich oder in anderer Kombination zur Ausgestaltung der Erfindung beitragen.

Die erfindungsgemäße Beleuchtungseinrichtung ist mit einer LED als Lichtquelle und mit einem Lichtleiter zur Weiterleitung des von der LED emittierten Lichts ausgestattet. Der Lichtleiter ist typischerweise vor einer lichtemittierenden Fläche der LED, das heißt typischerweise in Hauptausbreitungsrichtung des von der LED emittierten Lichts vor der LED angeordnet. Bei dem Lichtleiter kann es sich sowohl um einen einzelnen Stab aus einem Kern-Mantelglas als auch um ein Faserbündel handeln, dessen Fasern jeweils aus einem Kern-Mantelglas ausgebildet sind. Unter einem Kern-Mantelglas ist hierbei im üblichen Sinne ein aus zumindest zwei Glasarten bestehender Glaskörper zu verstehen, bei dem ein Kern aus einer ersten Glasart, dem Kernglas, von einem Mantel aus zumindest einer anderen Glasart, dem Mantelglas, umgeben ist. Zwischen der LED und dem Lichtleiter ist ein Lichtübertragungsteil angeordnet. Dieses Lichtübertragungsteil, welches für die von der LED emittierten Lichtstrahlen transparent ist, dient zur gezielten Einkopplung der von der LED emittierten Lichtstrahlen in den Lichtleiter.

Die erfindungsgemäße Beleuchtungseinrichtung zeichnet sich dadurch aus, dass das Lichtübertragungsteil zwei Abschnitte aufweist, von denen ein erster Abschnitt direkt der LED zugewandt ist und einen sich mit zunehmendem Abstand von der LED erweiternden Querschnitt sowie eine die in den ersten Abschnitt eintretende Strahlung winkelunabhängig reflektierende Mantelfläche aufweist und ein zweiter Abschnitt direkt dem Lichtleiter zugewandt ist und zumindest eine die von dem ersten Abschnitt in den zweiten Abschnitt eintretende Strahlung winkelabhängig reflektierende Mantelfläche aufweist. Dabei muss nicht zwangsweise die gesamte Mantelfläche des ersten Abschnitts für die in diesen eintretende Strahlung winkelunabhängig reflektierend sein und nicht zwangsweise die gesamte Mantelfläche des zweiten Abschnitts für die von dem ersten Abschnitt in den zweiten Abschnitt eintretende Strahlung winkelabhängig reflektierend sein. Nachfolgend wird jedoch im Sinne einer vereinfachten Beschreibung und Erläuterung immer nur der Fall betrachtet, dass die gesamte Mantelfläche des ersten Abschnitts für die in diesen eintretende Strahlung winkelunabhängig reflektierend sei, ohne sich dabei auf diesen speziellen Fall zu beschränken und dass die gesamte Mantelfläche des zweiten Abschnitts für die von dem ersten Abschnitt in den zweiten Abschnitt eintretende Strahlung winkelabhängig reflektierend sei, wobei es sich auch hier um keine Beschränkung auf diesen speziellen Fall handelt.

Der erste Abschnitt des Lichtübertragungsteils, welcher direkt der LED zugewandt ist, weist an seinem von der LED abgewandten Ende zweckmäßigerweise einen Querschnitt auf, welcher in etwa mit dem Querschnitt des Lichtleiters korrespondiert. Gegenüber diesem Querschnitt ist der Querschnitt des ersten Abschnitts an seinem der LED zugewandten Ende deutlich kleiner. Hierbei ist bei der Dimensionierung dieses Querschnitts allerdings sicherzustellen, dass das Lichtübertragungsteil an dem der LED zugewandten Ende ein Maximum der von der LED emittierten Lichtstrahlen aufnehmen kann.

Ein Teil dieser in den ersten Abschnitt des Lichtübertragungsteils eintretenden Lichtstrahlen weist einen vergleichsweise großen Winkel zu der Mittelachse des Lichtübertragungsteils auf und trifft somit auf die Mantelfläche, welche den ersten Abschnitt des Lichtübertragungsteils außenumfänglich begrenzt. Aufgrund der Eigenschaft dieser Mantelfläche, Lichtstrahlen winkelunabhängig zu reflektieren, geht an der Mantelfläche keiner der darauf auftreffenden Lichtstrahlen verloren, stattdessen werden all diese Lichtstrahlen mittels Reflexion weiter zu dem zweiten Abschnitt des Lichtübertragungsteils übertragen. Hierbei bewirkt die Formgebung des ersten Abschnitts des Lichtübertragungsteils mit dem sich in Richtung des zweiten Abschnitts des Lichtübertragungsteils kontinuierlich vergrößernden Querschnitt, dass die von der Mantelfläche reflektierten Lichtstrahlen einen Winkel relativ zu der Mittelachse des Lichtübertragungsteils aufweisen, welcher geringer als derjenige ist, den sie beim Eintritt in den ersten Abschnitt des Lichtübertragungsteils hatten. Durch diese in dem ersten Abschnitt des Lichtübertragungsteils an dessen Mantelfläche erfolgende Winkeltransformation wird der Anteil der von der LED emittierten Lichtstrahlen vergrößert, welche in dem auf das Lichtübertragungsteil folgenden Lichtleiter aufgrund dessen vergleichsweise geringen numerischen Apertur bzw. aufgrund der geringen numerischen Apertur der dort eingesetzten Fasern übertragen werden können. Hierbei ist der als Aspektverhältnis bezeichnete Quotient aus der Lichteintrittsfläche und der Lichtaustrittsfläche des ersten Abschnitts entscheidend, da ein minimales Aspektverhältnis eine maximale Winkeltransformation bewirkt. Allerdings wird eine Minimierung des Aspektverhältnisses dadurch eingeschränkt, dass, wie bereits angemerkt, bei der Dimensionierung des Querschnitts an dem der LED zugewandten Ende des ersten Abschnitts zu berücksichtigen ist, dass das Lichtübertragungsteil an dem der LED zugewandten Ende ein Maximum der von der LED emittierten Lichtstrahlen aufnehmen soll und der Querschnitt an dem von der LED abgewandten Ende in etwa mit dem Querschnitt des Lichtleiters korrespondieren soll. Trotz dieser Einschränkung werden mit der erfindungsgemäßen Beleuchtungseinrichtung aber ausgangsseitig des Lichtleiters deutlich höhere Lichtströme als bei vergleichbaren Beleuchtungseinrichtungen aus dem Stand der Technik erzielt.

Ungeachtet der in dem ersten Abschnitt des Lichtübertragungsteils stattfindenden Winkeltransformation können ausgangsseitig des ersten Abschnitts in Abhängigkeit von dem gewählten Aspektverhältnis immer noch einige Lichtstrahlen mit einem bezüglich der Mittelachse des Lichtübertragungsteils so großen Winkel vorliegen, dass diese Lichtstrahlen aufgrund der vergleichsweise geringen numerischen Apertur des Lichtleiters bzw. wegen der geringen numerischen Apertur der Einzelfasern des Lichtleiters nicht bis zu dem von dem Lichtübertragungsteil abgewandten Ende des Lichtleiters übertragen werden können. Da diesen Lichtstrahlen somit bezüglich der Lichtausbeute an dem von dem Lichtübertragungsteil abgewandten Ende des Lichtleiters keine Bedeutung zukommt und diese Lichtstrahlen sogar eine thermische Belastung des Lichtleiters verursachen und so zu einer Beschädigung des Lichtleiters führen können, kommt dem zweiten Abschnitt des Lichtübertragungsteils, welcher über seine gesamte Länge in Richtung der Mittelachse des Lichtübertragungsteils einen konstanten Querschnitt aufweist und somit stabförmig ausgebildet ist, die Aufgabe zu, diese Lichtstrahlen zu eliminieren. Zu diesem Zweck weist der zweite Abschnitt des Lichtübertragungsteils zumindest eine Mantelfläche auf, an welcher nur solche darauf auftreffende Lichtstrahlen reflektiert werden, die aufgrund der beschränkten numerischen Apertur des Lichtleiters bzw. der beschränkten numerischen Apertur der Fasern des Lichtleiters von dem Lichtleiter übertragen werden können, wohingegen Lichtstrahlen, welche einen relativ zu der Mittelachse des Lichtübertragungsteils großen Winkel aufweisen und von dem Lichtleiter nicht übertragen werden können, bereits den zweiten Abschnitt des Lichtübertragungsteils an dem Ort verlassen, an dem sie auf die Mantelfläche auftreffen.

Generell sollte das Lichtübertragungsteil möglichst nah an der LED angeordnet sein, damit ein möglichst großer Teil des von der LED emittierten Lichts in den ersten Abschnitt des Lichtübertragungsteils gelangen kann. Allerdings ist es auch von Vorteil, wenn sich zwischen der LED und dem Lichtübertragungsteil ein Medium befindet, welches einen Brechungsindex aufweist, der geringer als der Brechungsindex des Materials ist, aus welchem der erste Abschnitt des Lichtübertragungsteils ausgebildet ist, so dass die von der LED emittierten Lichtstrahlen beim Eintritt in den ersten Abschnitt des Lichtübertragungsteils möglichst stark in Richtung der Mittelachse des Lichtübertragungsteils gebrochen werden. Insofern ist gemäß einer vorteilhaften Weiterbildung der Erfindung vorgesehen, dass das Lichtübertragungsteil beabstandet von der LED angeordnet ist und ein Zwischenraum zwischen der LED und dem Lichtübertragungsteil gasgefüllt ist. Hierbei sollte der Abstand zwischen der LED und dem Lichtübertragungsteil im Hinblick auf die Lichtausbeute des Lichtübertragungsteils zweckmäßigerweise möglichst klein gehalten werden. Der dann vergleichsweise schmale Zwischenraum zwischen der LED und dem Lichtübertragungsteil ist vorteilhaft mit Luft oder einem anderen Gas gefüllt, dessen Brechungsindex in etwa n=1 beträgt. Da der erste Abschnitt des Lichtübertragungsteils vorzugsweise aus Glas, Kristall oder einem für die von der LED emittierten Lichtstrahlen transparenten Kunststoff ausgebildet ist und einen Brechungsindex aufweist, der deutlich größer ist, wird das von der LED emittierte Licht in ausreichendem Maße in Richtung der Mittelachse des Lichtübertragungsteils gebrochen.

Die Mantelfläche des ersten Abschnitts des Lichtübertragungsteils ist poliert und außenseitig von einem gasförmigen Medium umgeben. Dieser Ausgestaltung liegt der Umstand zugrunde, dass dann, wenn der erste Abschnitt des Lichtübertragungsteils, wie bereits erwähnt, aus einem Material wie Glas, Kristall oder einem für die von der LED emittierten Lichtstrahlen transparenten Kunststoff mit einem Brechungsindex von n=1,4 oder größer ausgebildet ist und der Brechungsindex des den ersten Abschnitt umgebenden Mediums, wie bei Luft und anderen gasförmigen Medien, in etwa n=1 beträgt, an der Mantelfläche des ersten Abschnitts die Bedingungen für eine Totalreflexion erfüllt sind. Mit der Polierung der Mantelfläche wird hierbei der Zweck verfolgt, dass die auf die Mantelfläche treffenden Lichtstrahlen an der Mantelfläche gerichtet reflektiert werden. Wegen des vergleichsweise großen Brechungsindexsprungs zwischen dem den ersten Abschnitt des Lichtübertragungsteils umgebenden Medium und dem Material des ersten Abschnitts des Lichtübertragungsteils und der Polierung der Mantelfläche werden im Wesentlichen alle von der LED in den ersten Abschnitt des Lichtübertragungsteils eintretenden Lichtstrahlen vergleichsweise stark in Richtung der Mittelachse des Übertragungsteils gebrochen und an der Mantelfläche des ersten Abschnitts des Übertragungsteils totalreflektiert.

Um eine zum Beispiel durch Stoffablagerungen verursachte unerwünschte zeit - und/oder umgebungs- bzw. einsatzbedingte Veränderung der Mantelfläche des ersten Abschnitts des Lichtübertragungsteils und eine daraus resultierende Einschränkung oder Störung der Totalreflexion zu verhindern, kann der erste Abschnitt des Lichtübertragungsteils vorteilhaft von einer Hülse umgeben sein, welche zweckmäßigerweise von der Mantelfläche des ersten Abschnitts beabstandet angeordnet ist und den ersten Abschnitt des Lichtübertragungsteil von dessen Außenumgebung abschirmt.

Beispielsweise dann, wenn man auf die vorgenannte umgebende Hülse verzichten will, kann es von Vorteil sein, wenn die Mantelfläche des ersten Abschnitts des Lichtübertragungsteils nicht nur poliert ist, sondern auch außenseitig der Mantelfläche eine für die von der LED emittierte Strahlung hoch reflektierende Schicht vorgesehen ist. Die hoch reflektierende Schicht, welche bei Verwendung einer Weißlicht emittierenden LED beispielsweise aus Silber oder Aluminium bestehen kann, bildet eine Spiegelfläche und dient hier zur Reflexion aller auf die Mantelfläche des ersten Abschnitts auftreffenden Lichtstrahlen. Auch hier wird mit der Polierung der Mantelfläche der Zweck verfolgt, dass die auf die Mantelfäche auftreffenden Lichtstrahlen an der Mantelfläche gerichtet reflektiert werden. Die Verwendung einer hoch reflektierenden Schicht an der Mantelfläche des ersten Abschnitts des Lichtübertragungsteils hat zudem den Vorteil, dass Ablagerungen an der Mantelfläche im Hinblick auf die Funktionsweise des ersten Abschnitts des Lichtübertragungsteils ohne Bedeutung sind, so lange sie nicht die hoch reflektierende Schicht verändern bzw. beschädigen. Eine den ersten Abschnitt des Lichtübertragungsteils außenseitig umgebende Hülse ist somit nicht erforderlich.

Der zweite Abschnitt des Lichtübertragungsteils ist gemäß der Erfindung ebenfalls aus einem Kern-Mantelglas gebildet. Das heißt, der zweite Abschnitt des Lichtübertragungsteils kann einen stabförmigen Kern aus einem Kernglas aufweisen, welcher an seinem Außenumfang von einem sich von dem Kernglas hinsichtlich des Brechungsindexes unterscheidenden Glas, dem Mantelglas, ummantelt ist. Das Kernglas und Mantelglas des zweiten Abschnitts des Lichtübertragungsteils stimmen zweckdienlich mit dem in dem Lichtleiter verwendeten Kernglas und Mantelglas überein oder weisen einen Brechungsindexsprung auf, welcher im Wesentlichen mit dem Brechungsindexsprung zwischen dem Brechungsindex des in dem Lichtleiter verwendeten Kernglases und dem Brechungsindex des in dem Lichtleiter verwendeten Mantelglases übereinstimmt. Der Brechungsindexsprung zwischen dem Kernglas und dem Mantelglas, welche in dem zweiten Abschnitt des Lichtübertragungsteils verwendet werden, legt fest, welche Lichtstrahlen aufgrund von Totalreflexion weiter zu dem Lichtleiter übertragen werden und welche Lichtstrahlen den zweiten Abschnitt des Lichtübertragungsteils bereits an seiner Mantelfläche verlassen, weil die Voraussetzung für ein Totalreflexion nicht mehr erfüllt sind.

Bei einem aus einem Kern-Mantelglas ausgebildeten zweiten Abschnitt des Lichtübertragungsteils verlassen bei einer ausreichend großen Länge des zweiten Abschnitts in Richtung der Mittelachse des Lichtübertragungsteils Lichtstrahlen, deren Winkel gegenüber der Mittelachse des Lichtübertragungsteils groß genug ist, das Kernglas des zweiten Abschnitts und dringen in dessen Mantelglas ein, weil an dem Übergang von Kernglas zu Mantelglas nicht mehr die Bedingungen für eine Totalreflexion erfüllt sind. Insbesondere dann, wenn das Mantelglas außenseitig von einem gasförmigen Medium mit einem Brechungsindex von in etwa n=1 umgeben ist und das Mantelglas eine glatte äußere Mantelfläche aufweist, kann der dann vergleichsweise große Brechungsindexsprung zwischen dem Brechungsindex des Mantelglases und dem Brechungsindex des gasförmigen Mediums zur Folge haben, dass an der Schnittstelle zwischen dem Mantelglas und dessen äußerer Umgebung eine Totalreflexion der in das Mantelglas eingedrungenen Lichtstrahlen stattfindet. Die in das Mantelglas eingedrungene Lichtstrahlen werden dann in unerwünschter Weise innerhalb des Mantelglases zu dem Lichtleiter weitergeleitet. Um dies zu verhindern, ist bevorzugt vorgesehen, dass die äußere Mantelfläche des Mantelglases aufgeraut ist. Durch diese Maßnahme verlassen die in das Mantelglas eingedrungenen Lichtstrahlen das Mantelglas spätestens nach einigen Reflexionen an der Schnittstelle zwischen dem Mantelglas und dessen äußerer Umgebung und gelangen über die äußere Mantelfläche in die äußere Umgebung des zweiten Abschnitts des Lichtübertragungsteils.

Anstelle eines aus Kern-Mantelglas ausgebildeten zweiten Abschnitts des Lichtübertragungsteils, kann der zweite Abschnitt des Lichtübertragungsteils vorteilhaft auch lediglich aus einem für die von der LED emittierten Lichtstrahlen transparenten Material ausgebildet sein, wobei der zweite Abschnitt dann an seiner Mantelfläche von einem Material umgeben ist, dessen Brechungsindex sich von dem Brechungsindex des Materials des zweiten Abschnitts des Lichtübertragungsteils unterscheidet. Um bei dieser Ausgestaltung Lichtstrahlen mit einem großen Winkel relativ zu der Mittelachse des Lichtübertragungsteils in dem zweiten Abschnitt des Lichtübertragungsteils eliminieren zu können, sind das Material des zweiten Abschnitts und das diesen Abschnitt außenumfänglich umgebende Material zweckmäßigerweise so gewählt, dass das Material des zweiten Abschnitts und das diesen Abschnitt umgebende Material den gleichen Brechungsindexsprung wie das Kern-Mantelglas des Lichtleiters erzeugt. Der zweite Abschnitt des Lichtübertragungsteils kann zum Beispiel aus reinem Kernglas ausgebildet sein. Bei dem Material, welches den zweiten Abschnitt umgibt, kann es sich um einen Klebstoff handeln, der auch dazu verwendet werden kann, den zweiten Abschnitt und vorzugsweise das gesamte Lichtübertragungsteil in einer Einbauumgebung zu fixieren. Ferner kann es unter gewissen Umständen auch von Vorteil sein, wenn das den zweiten Abschnitt umgebende Material für die von der LED emittierten Lichtstrahlen transparent ausgebildet ist. Aufgrund der Transparenz des Materials wird von diesem praktisch keine Strahlung absorbiert, die ansonsten gegebenenfalls die Alterung dieses Materials beschleunigen würde. Dies kann insbesondere dann von Bedeutung sein, wenn es sich bei dem den zweiten Abschnitt des Lichtübertragungsteils umgebenden Material um einen Kunststoff, wie beispielsweise einen Klebstoff handelt, und in der Beleuchtungseinrichtung hohe Strahlungsleistungen übertragen werden sollen.

Gemäß einer weiteren bevorzugten Weiterbildung der Erfindung ist der zweite Abschnitt des Lichtübertragungsteils bevorzugt von einem Bauteil umgeben, welches zur Aufnahme der von der LED emittierten Strahlung ausgebildet ist. Demnach ist das den zweiten Abschnitt umgebende Bauteil, bei dem es sich zweckmäßigerweise um einen Hülse handelt, vorteilhaft derart ausgebildet, dass es ein großes Absorptionsvermögen für die von der LED emittierten Lichtstrahlen hat und vorteilhafterer Weise auch eine hohe Wärmeleitfähigkeit besitzt. So kann das Bauteil beispielsweise aus Metall ausgebildet sein und an seiner dem zweiten Abschnitt des Lichtübertragungsteils zugewandten Seite beispielsweise mit einer Oxalschicht nicht leitend oberflächenbeschichtet sein. Ferner kann das Bauteil mit dem zweiten Abschnitt des Lichtübertragungsteils über einen die Mantelfläche des zweiten Abschnitts vollständig umgebenden Klebstoff verbunden sein. Hierbei erweist es sich als günstig, wenn der Klebstoff eine hohe Transparenz für die von der LED emittierten Lichtstrahlen aufweist. Wenn der zweite Abschnitt von einem Kern-Mantelglas gebildet wird, ist es zudem sinnvoll, wenn der Brechungsindex des transparenten Klebstoffes weitestgehend dem Brechungsindex des Mantelglases des Kern-Mantelglases entspricht.

Als Alternative zu einer Ausgestaltung, bei welcher der zweite Abschnitt des Lichtübertragungsteils von einem stabartigen Bauteil aus Kern-Mantelglas gebildet wird, kann der zweite Abschnitt des Lichtübertragungsteils auch von einem Faserbündel gebildet werden, wobei die Fasern des Faserbündels jeweils aus Kern-Mantelglas bestehen. Hierbei ist der Brechungsindexsprung zwischen dem Kernglas und dem Mantelglas der einzelnen Fasern des zweiten Abschnitts idealerweise im Wesentlichen gleich dem Brechungsindexsprung zwischen dem Kernglas und dem Mantelglas des Lichtleiters. Ein Vorteil eines von einem Faserbündel gebildeten zweiten Abschnitts des Lichtübertragungsteils besteht darin, dass der Querschnitt des zweiten Abschnitts vergleichsweise einfach an den Querschnitt eines ebenfalls aus einem Faserbündel bestehenden Lichtleiters angepasst werden kann.

Im einfachsten Fall wird das Lichtübertragungsteil vorteilhaft von einem Bauteil gebildet. So kann als Basisteil für das Lichtübertragungsteil ein Stab aus Kern-Mantelglas dienen, dessen Querschnitt im Wesentlichen mit dem Querschnitt des Lichtleiters übereinstimmt. Zur Herstellung des Lichtübertragungsteils aus dem Basisteil wird an einem an ein Ende des Basisteils angrenzenden Bereich des Basisteils das Mantelglas und auch ein Teil des Kernglases derart entfernt, dass sich der Querschnitt des Basisteils ausgehend von dem Bereich, an dem das Basisteil noch das Mantelglas aufweist, verjüngt.

Anstelle einer einteiligen Ausgestaltung des Übertragungsteils ist erfindungsgemäß auch vorgesehen, dass das Lichtübertragungsteil von zwei separaten Bauteilen gebildet wird. In diesem Fall bildet ein Bauteil den der LED direkt zugewandten ersten Abschnitt des Lichtübertragungsteils, welcher einen sich mit zunehmendem Abstand von der LED erweiternden Querschnitt sowie eine die in den ersten Abschnitt eintretende Strahlung winkelunabhängig reflektierende Mantelläche aufweist, und ein zweites Bauteil den direkt dem Lichtleiter zugewandten zweiten Abschnitt des Lichtübertragungsteils, welcher zumindest eine die von dem ersten Abschnitt in den zweiten Abschnitt eintretende Strahlung winkelabhängig reflektierende Mantelfläche aufweist. Die beiden das Lichtübertragungsteil bildenden Bauteile können sowohl lose nebeneinander angeordnet sein oder miteinander verbunden sein. Hierbei ist auf jeden Fall sicherzustellen, dass die beiden Bauteile möglichst nah zueinander positioniert sind, um Übertragungsverluste beim Übergang der Lichtstrahlen von dem ersten Bauteil zu dem zweiten Bauteil zu minimieren. Im Hinblick auf solche Übertragungsverluste ist es bei einer Ausgestaltung, bei welcher die beiden Bauteile miteinander verbunden sind, sinnvoll, wenn zur Verbindung der beiden Bauteile ein ausreichend temperaturbeständiger optischer Klebstoff verwendet wird, wobei der eingesetzte Klebstoff besonders vorteilhaft einen Brechungsindex aufweist, welcher weitestgehend mit dem Brechungsindex der Materialien der beiden Bauteile übereinstimmt, welche dazu dienen, die von der LED emittierten Lichtstrahlen zu dem Lichtleiter zu überführen.

Es ist bereits darauf hingewiesen worden, dass der Querschnitt des ersten Abschnitts des Lichtübertragungsteils einerseits groß genug ausgebildet sein sollte, um ein Maximum der von der LED emittierten Lichtstrahlen aufnehmen zu können und andererseits möglichst kleingehalten werden sollte, um ein möglichst günstiges Aspektverhältnis zu erlangen. In diesem Zusammenhang ist vorteilhaft vorgesehen, dass das Querschnittsprofil des ersten Abschnitts des Lichtübertragungsteils an dem der LED zugewandten Ende des ersten Abschnitts mit dem Profil der lichtemittierenden Fläche der LED in lateraler Richtung korrespondiert. Das heißt, der Querschnitt des ersten Abschnitts des Lichtübertragungsteils stimmt hinsichtlich seiner Größe und Form vorzugsweise im Wesentlichen mit der lichtemittierenden Fläche der LED in lateraler Richtung überein. Da die lichtemittierenden Flächen von LED-Chips meist rechteckig oder quadratisch ausgebildet sind, weist somit auch der Querschnitt des ersten Abschnitts des Lichtübertragungsteils vorrangig an dessen der LED zugewandten Ende einen rechteckigen oder quadratischen Querschnitt mit den Abmessungen der lichtemittierenden Fläche der LED auf. Sollte die lichtemittierende Fläche der LED einen runden Querschnitt aufweisen, weist auch der Querschnitt des ersten Abschnitts des Lichtübertragungsteils an dessen der LED zugewandten Ende einen entsprechend dimensionierten runden Querschnitt auf.

Bei Anwendungen, bei welchen es nicht erforderlich ist, den Lichtleiter aus der erfindungsgemäßen Beleuchtungseinrichtung zu entnehmen, erweist es sich als vorteilhaft, wenn das Lichtübertragungsteil mit dem Lichtleiter mittels einer temperaturbeständigen stoffschlüssigen Verbindung verbunden ist. In diesem Zusammenhang wird zur stoffschlüssigen Verbindung des Lichtübertragungsteils mit dem Lichtleiter zwischen dem zweiten Abschnitt des Lichtübertragungsteils und dem Lichtleiter vorteilhaft ein temperaturbeständiger optischer Klebstoff eingesetzt, wobei es hinsichtlich der Übertragungseffizienz von dem Lichtübertragungsteil zu dem Lichtleiter sinnvoll ist, wenn der verwendete Klebstoff einen Brechungsindex aufweist, welcher weitestgehend mit dem Brechungsindex der Materialien der beiden Bauteile übereinstimmt, welche dazu dienen, die von der LED emittierten Lichtstrahlen von dem Lichtübertragungsteil zu dem Lichtleiter zu überführen.

Nachfolgend ist die Erfindung anhand von in der Zeichnung dargestellten Ausführungsbeispielen näher erläutert. In der Zeichnung zeigt jeweils schematisch vereinfacht und in unterschiedlichen Maßstäben:
- Fig. 1: in einer Seitenansicht eine Beleuchtungseinrichtung mit einem Lichtübertragungsteil,
- Fig. 2: in einer Seitenansicht ein Lichtübertragungsteils gemäß einer zweiten Ausgestaltung,
- Fig. 3: in einer Seitenansicht ein Lichtübertragungsteil gemäß einer dritten Ausgestaltung,
- Fig. 4: in einer Seitenansicht das Lichtübertragungsteil nach Fig. 3,
- Fig. 5: in einer Seitenansicht ein Lichtübertragungsteil gemäß einer vierten Ausgestaltung,
- Fig. 6: in einer Seitenansicht ein Lichtübertragungsteil gemäß einer fünften Ausgestaltung,
- Fig. 7: in einer Seitenansicht ein Lichtübertragungsteil gemäß einer sechsten Ausgestaltung,
- Fig. 8: in einer Seitenansicht ein Lichtübertragungsteil gemäß einer siebten Ausgestaltung,
- Fig. 9: in einer Seitenansicht ein Lichtübertragungsteil und einen Lichtleiter,
- Fig. 10: in einer Seitenansicht ein Lichtübertragungsteil gemäß einer achten Ausgestaltung,
- Fig. 11: in einer Seitenansicht ein Lichtübertragungsteil gemäß einer neunten Ausgestaltung und
- Fig. 12: in einer Seitenansicht ein Lichtübertragungsteil und ein Lichtleitkabel.

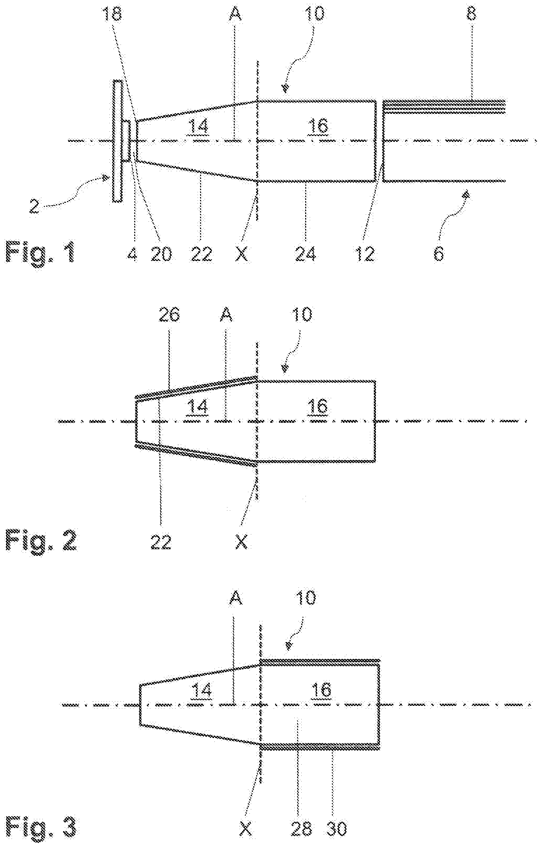

Die in Fig. 1 dargestellte Beleuchtungseinrichtung weist neben einer LED 2 mit einer lichtemittierenden Fläche 4 einen Lichtleiter 6 auf, in den das von der LED 2 emittierte Licht eingekoppelt wird. Der Lichtleiter 6 besteht aus einer Vielzahl lichtleitender Fasern 8, welche jeweils aus Kern-Mantelglas bestehen. Der Querschnitt einer der LED 2 zugewandten Stirnfläche 12 des Lichtleiters 6 ist größer als der Querschnitt der lichtemittierenden Fläche 4 der LED 2. Die Einkopplung der von der LED 2 emittierten Lichtstrahlen in den Lichtleiter 6 erfolgt nicht direkt sondern über ein zwischen der LED 2 und dem Lichtleiter 6 angeordnetes Lichtübertragungsteil 10, welches aus einem für die von der LED 2 emittierte Strahlung transparenten Material mit einem Brechungsindex n>1,4 besteht.

Wie in der Zeichnung durch die Strichlinie X kenntlich gemacht, weist das Lichtübertragungsteil 10 zwei Abschnitte 14 und 16 auf. Von diesen beiden Abschnitten 14 und 16 ist ein erster Abschnitt 14 direkt der LED 2 zugewandt und ein zweiter Abschnitt 16 direkt dem Lichtleiter 6 zugewandt. Die äußere Form des zweiten Abschnitts 16 des Lichtübertragungsteils 10 ist zylindrisch, wobei der Querschnitt des zweiten Abschnitts 16 dem Querschnitt der Stirnfläche 12 des Lichtleiters 6 entspricht.

Ausgehend von dem Querschnitt des zweiten Abschnitts 16 verringert sich der Querschnitt des ersten Abschnitts 14 des Lichtübertragungsteils 10 kontinuierlich mit geringer werdendem Abstand zu der LED 2. So weist eine der lichtemittierenden Fläche 4 der LED 2 unmittelbar gegenüberliegende Stirnfläche 18 einen geringeren Querschnitt als eine an der Strichlinie X befindliche Stirnfläche auf. Um möglichst viel von der Strahlung aufnehmen zu können, welche von der LED 2 emittiert wird, entspricht der Querschnitt der Stirnfläche 18 des ersten Abschnitts 14 des Lichtübertragungsteils 10 zumindest dem Querschnitt der lichtemittierenden Fläche 4 der LED 2, wobei der Abstand zwischen der Stirnfläche 18 des ersten Abschnitts 14 des Übertragungsteils 10 und der lichtemittierenden Fläche 4 der LED 2 sehr gering ist. In einem Zwischenraum 20 zwischen der Stirnfläche 18 und der lichtemittierenden Fläche 4 der LED 2 befindet sich Luft, welches in dem in Fig. 1 dargestellten Ausführungsbeispiel auch das gesamte Lichtübertragungsteil 10 außenseitig umgibt und einen Brechungsindex von n=1 aufweist.

Ein Teil der von der LED 2 emittierten Lichtstrahlen weist einen vergleichsweise großen Winkel zu einer Mittelachse A des Lichtübertragungsteils 10 auf. Diese Lichtstrahlen treffen in dem ersten Abschnitt 14 des Lichtübertragungsteils 10 auf eine äußere, polierte Mantelfläche 22 des Abschnitts 14. Aufgrund des verhältnismäßig großen Brechungsindexsprungs zwischen dem Material, aus dem der Abschnitt 14 des Lichtübertragungsteils 10 besteht, und der den Abschnitt 14 umgebenden Luft, werden die auf die Mantelfläche 22 des Abschnitts 14 treffenden Strahlen unabhängig von ihrem Winkel zu der Mantelfläche 22 an der Mantelfläche 22 totalreflektiert. Die Formgebung des ersten Abschnitts 14 des Lichtübertragungsteils 10 mit dem sich in Richtung des zweiten Abschnitts 16 des Lichtübertragungsteils 10 kontinuierlich vergrößernden Querschnitt bewirkt hierbei, dass die von der Mantelfläche 22 reflektierten Strahlen einen Winkel relativ zu der Mittelachse A des Lichtübertragungsteils 10 aufweisen, welcher geringer als derjenige ist, den sie beim Eintritt in den ersten Abschnitt 14 des Lichtübertragungsteils 10 hatten.

Trotz dieser Winkeltransformation können ausgangsseitig des ersten Abschnitts 14 des Lichtübertragungsteils 10 noch einige Lichtstrahlen mit einem bezüglich der Mittelachse A des Lichtübertragungsteils 10 so großen Winkel vorliegen, dass diese Lichtstrahlen aufgrund der vergleichsweise geringen numerischen Apertur der lichtleitenden Fasern 8 des Lichtleiters 6 nicht bis zu dem von dem Lichtübertragungselement 10 abgewandten Ende des Lichtleiters 6 übertragen werden können. Diese Lichtstrahlen werden in dem zweiten Abschnitt 16 des Lichtübertragungsteils 10 eliminiert. Zu diesem Zweck weist der zweite Abschnitt 16 des Lichtübertragungsteils 10 bei dem in Fig. 1 dargestellten Ausführungsbeispiel eine äußere Mantelfläche 24 auf, an welcher nur solche darauf auftreffende Lichtstrahlen reflektiert werden, die aufgrund der beschränkten numerischen Apertur der lichtleitenden Fasern 8 des Lichtleiters 6 von dem Lichtleiter 6 übertragen werden können. Die übrigen Lichtstrahlen, welche einen relativ zu der Mittelachse A des Lichtübertragungsteils 10 großen Winkel aufweisen und von dem Lichtleiter 6 nicht übertragen werden können, verlassen den zweiten Abschnitt 16 des Lichtübertragungsteils 10 über dessen Mantelfläche 24.

Das in Fig. 2 dargestellte Lichtübertragungsteil 10 unterscheidet sich von dem in Fig. 1 gezeigten Lichtübertragungsteil 10 hinsichtlich der Ausgestaltung des ersten Abschnitts 14 des Lichtübertragungsteils 10. Hier ist auf die Außenseite der äußeren Mantelfläche 22 des ersten Abschnitts 14 eine hoch reflektierende Schicht 26 aufgetragen. Diese Schicht 26 bildet somit eine Spiegelfläche, an der alle auf die Mantelfläche 22 des ersten Abschnitts 14 auftreffende Lichtstrahlen reflektiert werden.

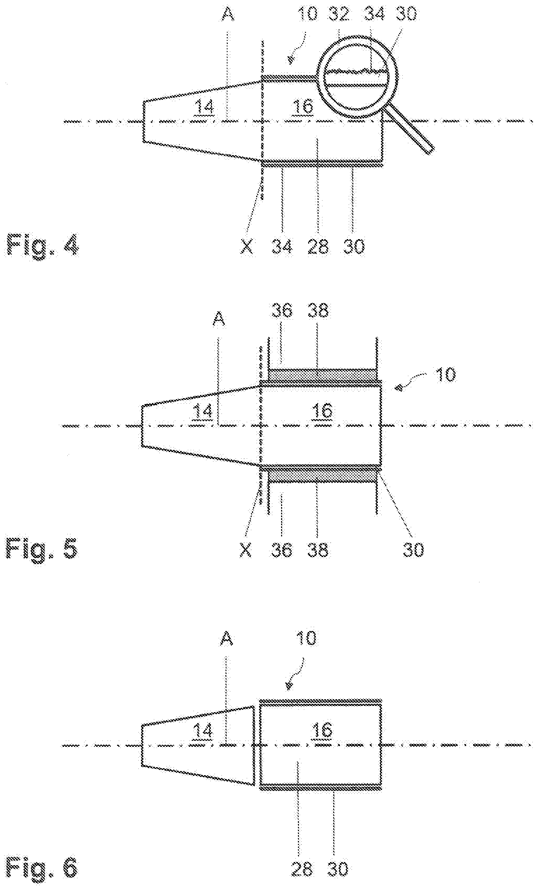

In den Fig. 3 und 4 ist ein Lichtübertragungsteil 10 dargestellt, dessen zweiter Abschnitt 16 aus einem Kern-Mantelglas ausgebildet ist. Hierbei weist der zweite Abschnitt 16 einen Kern 28 aus einem Kernglas auf. Außenumfänglich schließt sich an den Kern 28 ein Mantel 30 aus einem Mantelglas an, der die äußere Mantelfläche 34 des zweiten Abschnitts 16 bildet. Das verwendete Kernglas und Mantelglas des zweiten Abschnitts 16 stimmen mit dem Kernglas und Mantelglas überein, welches bei einem in Verbindung mit dem Lichtüberragungsteil 10 eingesetzten Lichtleiter 6 verwendet wird.

In Fig. 4 ist ein Bereich des Mantels 30 des zweiten Abschnitts 16 des Lichtübertragungsteils 10 vergrößert dargestellt und durch eine Lupe 32 kenntlich gemacht. Dem von der Lupe 32 eingefassten Bereich ist zu entnehmen, dass die äußere Mantelfläche 34 des Mantels 30 aus Mantelglas aufgeraut ist. Hierdurch soll eine Totalreflexion der in den Mantel 30 eingedrungenen Lichtstrahlen verhindert werden.

Fig. 5 ist eine Ausgestaltung zu entnehmen, bei welcher der zweite Abschnitt 16 des Lichtübertragungsteils 10 von einem Bauteil 36 umgeben ist. Der zweite Abschnitt 16 ist in Fig. 5 rein beispielhaft als Kern-Mantelglas mit einem Kern 28 und einem Mantel 30 dargestellt. Das Bauteil 36, bei dem es sich um eine Halterung für das Lichtübertragungsteil 10 handeln kann, ist aus Metall ausgebildet und besitzt aufgrund seiner Oberflächenbeschaffenheit, weil es beispielsweise eloxiert wurde, ein großes Absorptionsvermögen für die von der LED 2 emittierten Lichtstrahlen und eine hohe Wärmeleitfähigkeit. Über eine an der Außenseite des Mantels 30 aufgetragene Schicht aus Klebstoff 38 ist das Bauteil 36 mit dem zweiten Abschnitt 16 des Lichtübertragungsteils 10 stoffschlüssig verbunden. Dieser Klebstoff 38 weist eine hohe Transparenz für die von der LED 2 emittierten Lichtstrahlen auf und besitzt einen Brechungsindex, welcher weitestgehend dem Brechungsindex des Mantelglases des Kern-Mantelglases entspricht.

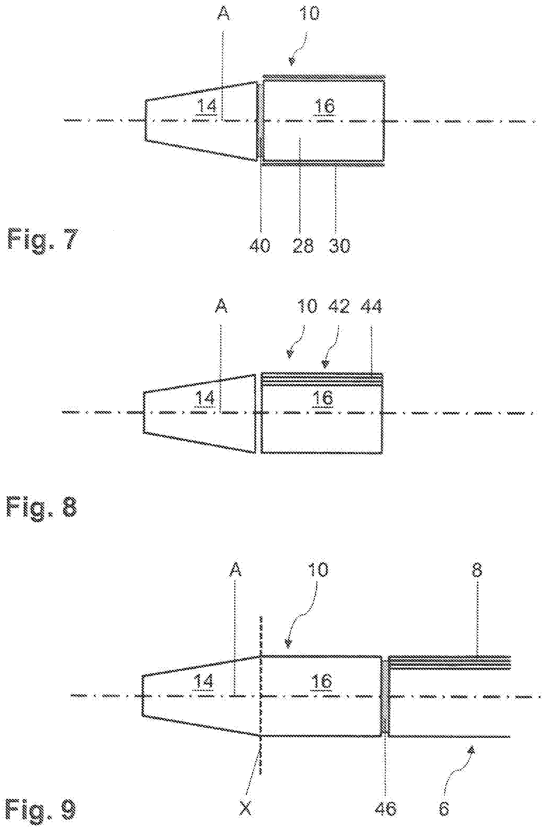

Während die in den Fig. 1 - 5 dargestellten Lichtübertragungsteile 10 einteilig ausgebildet sind, sind die in den Fig. 6 - 8 dargestellten Lichtübertragungsteile 10 zweiteilig ausgebildet. Hier werden der erste Abschnitt 14 und der zweite Abschnitt 16 des Lichtübertragungsteils 10 jeweils von separaten Bauteilen gebildet. Im Gegensatz zu den in den Fig. 6 und 8 gezeigten Lichtübertragungsteilen 10, bei denen die beiden, den ersten Abschnitt 14 und den zweiten Abschnitt 16 bildenden Bauteile lose nebeneinander angeordnet sind, ist bei dem in Fig. 7 dargestellten Lichtübertragungsteil 10 das Bauteil, welches den ersten Abschnitt 14 des Lichtübertragungsteils 10 bildet, stoffschlüssig mit dem den zweiten Abschnitt 16 des Lichtübertragungsteils 10 bildenden Bauteil verbunden. Hierzu ist zwischen dem den ersten Abschnitt 14 bildenden Bauteil und dem den zweiten Abschnitt 16 bildenden Bauteil eine Schicht aus einem temperaturbeständigen optischen Klebstoff 40 angeordnet. Der verwendete Klebstoff 40 weist einen Brechungsindex auf, der im Wesentlichen den Brechungsindizes der beiden Abschnitte 14 und 16 des Lichtübertragungsteils 10 entspricht.

Das in Fig. 8 dargestellte Lichtübertragungsteil 10 unterscheidet sich von dem in Fig. 6 dargestellten Lichtübertragungsteil 10 lediglich im Hinblick auf die Ausgestaltung des zweiten Abschnitts 16 des Lichtübertragungsteils 10. Im Gegensatz zu dem in Fig. 6 gezeigten Lichtübertragungsteil 10, bei dem der zweite Abschnitt 16 aus einem Kern-Mantelglas mit einem Kern 28 und einem Mantel 30 ausgebildet ist, wird der zweite Abschnitt 16 des in Fig. 8 dargestellten Lichtübertragungsteils 10 von einem Faserbündel 42 mit einer Vielzahl von Fasern 44 aus Kern-Mantelglas gebildet. Hierbei ist der Brechungsindexsprung zwischen dem Kernglas und dem Mantelglas der einzelnen Fasern 44 gleich dem Brechungsindexsprung zwischen dem Kernglas und dem Mantelglas der Fasern 8 des Lichtleiters 6.

Aus Fig. 9 geht hervor, dass das Lichtübertragungsteil 10 stoffschlüssig mit dem Lichtleiter 6 verbunden sein kann. Diese Verbindung geschieht durch eine Schicht aus Klebstoff 46, welche zwischen dem Lichtübertragungsteil 10 und dem Lichtleiter 6 angeordnet ist. Der verwendete Klebstoff 46 ist für die von der LED 2 emittierten Lichtstrahlen transparent sowie temperaturbeständig und weist einen Brechungsindex auf, welcher mit dem Brechungsindex der Fasern 8 des Lichtleiter 6 und dem Brechungsindex des Materials des Abschnitts 16 übereinstimmt, welches zur Weiterleitung der von der LED 2 emittierten Lichtstrahlen in den Lichtleiter 6 dient.

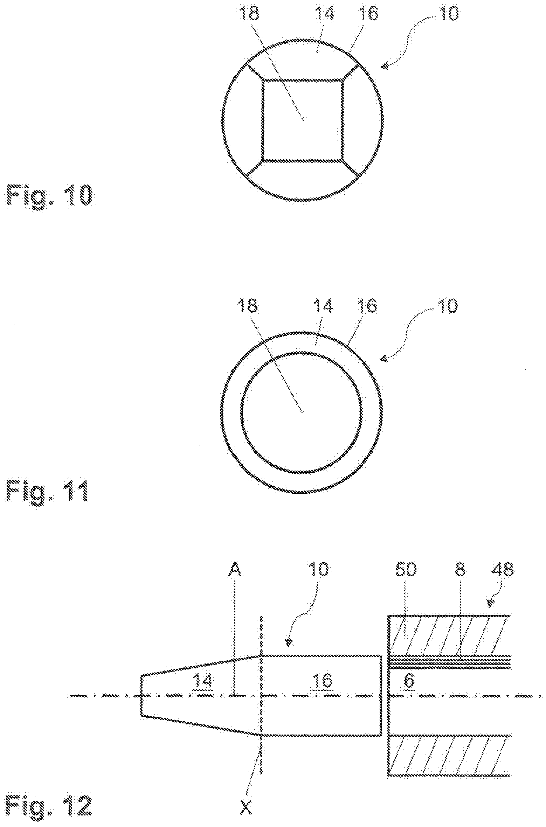

Der Querschnitt des ersten Abschnitts 14 des Lichtübertragungsteils 10 korrespondiert an seinem der LED 2 zugewandten Ende hinsichtlich seiner Form und Abmessungen mit dem Querschnitt der lichtemittierenden Fläche 4 der LED 2. Dies wird insbesondere aus den Fig. 10 und 11 deutlich. Weist die LED 2 eine rechteckige lichtemittierende Fläche 4 auf, so ist, wie in Fig. 10 dargestellt, die Stirnfläche 18 des ersten Abschnitts 14 des Lichtübertragungsteils 10 ebenfalls rechteckig mit den Abmessungen der lichtemittierenden Fläche 4 der LED 2 ausgebildet. In diesem Fall, weist der erste Abschnitt 14 eine pyramidenstumpfartige Form auf. Ist der Querschnitt der lichtemittierenden Fläche 4 der LED 2 rund, so ist die Stirnfläche 18 des ersten Abschnitts 14 des Lichtübertragungsteils 10 ebenfalls rund mit dem Durchmesser der lichtemittierenden Fläche 4 der LED 2 ausgebildet. In diesem Fall ist der erste Abschnitt 14 des Lichtübertragungsteils kegelstumpfförmig ausgebildet. Dies wird aus Fig. 11 deutlich.

Schließlich zeigt Fig. 12, dass ein aus einer Vielzahl lichtleitender Fasern 8 bestehender Lichtleiter 6 Teil eines Lichtleitkabels 48 sein kann und in diesem Fall in einer Hülse 50 angeordnet ist. Bei der Hülse 50 kann es sich z.B. um eine Metallhülse handeln.

### Bezugszeichenliste

- 2: LED
- 4: Lichtemittierende Fläche
- 6: Lichtleiter
- 8: Lichtleitende Faser
- 10: Lichtübertragungsteil
- 12: Stirnfläche
- 14: Abschnitt
- 16: Abschnitt
- 18: Stirnfläche
- 20: Zwischenraum
- 22: Mantelfläche
- 24: Mantelfläche
- 26: Schicht
- 28: Kern
- 30: Mantel
- 32: Lupe
- 34: Mantelfläche
- 36: Bauteil
- 38: Klebstoff
- 40: Klebstoff
- 42: Faserbündel
- 44: Faser
- 46: Klebstoff
- 48: Lichtleitkabel
- 50: Hülse

- A: Mittelachse
- X: Strichlinie

## Patentansprüche

1. Beleuchtungseinrichtung, mit einer LED (2), mit einem Lichtleiter (6) und mit einem zwischen der LED (2) und dem Lichtleiter (6) angeordneten Lichtübertragungsteil (10), welches zwei Abschnitte (14, 16) aufweist, von denen ein erster Abschnitt (14) direkt der LED (2) zugewandt ist und einen sich mit zunehmendem Abstand von der LED (2) erweiternden Querschnitt sowie eine die in den ersten Abschnitt (14) eintretende Strahlung weitestgehend winkelunabhängig reflektierende Mantelfläche (22) aufweist und von denen ein zweiter Abschnitt (16) direkt dem Lichtleiter (6) zugewandt ist, der einen in Richtung der Mittelachse des Lichtübertragungsteils konstanten Querschnitt aufweist und der zumindest eine die von dem ersten Abschnitt (14) in den zweiten Abschnitt (16) eintretende Strahlung weitestgehend winkelabhängig reflektierende Mantelfläche (24) aufweist, **dadurch gekennzeichnet,**
**dass** es sich bei dem Lichtleiter (6) um einen einzelnen Stab aus einem Kern-Mantelglas oder um ein Faserbündel von lichtleitenden Fasern (8) handelt, die jeweils aus einem Kern-Mantelglas bestehen,
**dass** zumindest ein Teil der Mantelfläche (22) des ersten Abschnittes (14) poliert und dadurch so ausgebildet ist, dass die Bedingungen für eine Totalreflektion erfüllt sind, und
**dass** zumindest ein Teil des zweiten Abschnitts (16) aus einem Kern-Mantelglas gebildet ist, wobei das Kernglas (28) und das Mantelglas (30) des zweiten Abschnitts (16) einen Brechungsindexsprung aufweisen, welcher mit dem Brechungsindexsprung zwischen dem Brechungsindex des in dem Lichtleiter (6) verwendeten Kernglases und dem Brechungsindex des in dem Lichtleiter (6) verwendeten Mantelglases übereinstimmt.

2. Beleuchtungseinrichtung nach Anspruch 1, bei welcher das Lichtübertragungsteil (10) beabstandet von der LED (2) angeordnet ist und ein Zwischenraum (20) zwischen der LED (2) und dem Lichtübertragungsteil (10) gasgefüllt ist.

3. Beleuchtungseinrichtung nach einem der vorangehenden Ansprüche, bei welcher die Mantelfläche (22) des ersten Abschnitts (14) des Lichtübertragungsteils (10) poliert ist und außenseitig von einem gasförmigen Medium umgeben ist.

4. Beleuchtungsvorrichtung nach einem der Ansprüche 1 oder 2, bei welcher die Mantelfläche (22) des ersten Abschnitts (14) des Lichtübertragungsteils (10) poliert ist, wobei außenseitig der Mantelfläche (22) eine für die von der LED (2) emittierte Strahlung hochreflektierende Schicht (26) vorgesehen ist.

5. Beleuchtungseinrichtung nach einem der Ansprüche 1 bis 4, bei welchem eine äußere Mantelfläche (34) des Mantelglases aufgeraut ist.

6. Beleuchtungseinrichtung nach einem der Ansprüche 1 bis 5, bei welchem der zweite Abschnitt (16) des Lichtübertragungsteils (10) aus einem für die von der LED (2) emittierte Strahlung transparenten Material ausgebildet ist und an seiner Mantelfläche von einem Material umgeben ist, dessen Brechungsindex sich von dem Brechungsindex des Materials des zweiten Abschnitts (16) des Lichtübertragungsteils (10) unterscheidet.

7. Beleuchtungseinrichtung nach einem der vorangehenden Ansprüche, bei welchem der zweite Abschnitt (16) des Lichtübertragungsteils (10) von einem Bauteil (36) umgeben ist, welches zur Aufnahme der von der LED (2) emittierten Strahlung ausgebildet ist.

8. Beleuchtungseinrichtung nach einem der vorangehenden Ansprüche, bei welchem der zweite Abschnitt (16) des Lichtübertragungsteils (10) von einem Faserbündel (42) gebildet wird, wobei die Fasern (44) des Faserbündels (42) jeweils aus Kern-Mantelglas bestehen.

9. Beleuchtungseinrichtung nach einem der vorangehenden Ansprüche, bei welchem das Lichtübertragungsteil (10) von einem Bauteil gebildet wird.

10. Beleuchtungseinrichtung nach einem der vorangehenden Ansprüche, bei welchem das Lichtübertragungsteil (10) von zwei separaten Bauteilen gebildet wird.

11. Beleuchtungseinrichtung nach einem der vorangehenden Ansprüche, bei welchem das Querschnittsprofil des ersten Abschnitts (14) des Lichtübertragungsteils (10) an dem der LED (2) zugewandten Ende des ersten Abschnitts (14) mit dem Profil der lichtemittierenden Fläche (4) der LED (2) in lateraler Richtung korrespondiert.

12. Beleuchtungseinrichtung nach einem der vorangehenden Ansprüche, bei welchem das Lichtübertragungsteil (10) mit dem Lichtleiter (6) mittels einer temperaturbeständigen stoffschlüssigen Verbindung verbunden ist.

## Claims

1. An illumination device, with an LED (2), with a fibre-optic (6) and with a light transmission part (10) which is arranged between the LED (2) and the fibre-optic (6) and which comprises two sections (14, 16), of which a first section (14) directly faces the LED (2) and has a cross section which widens with an increasing distance to the LED (2) and well as has a lateral surface (22) which in a largely angularly independent manner reflects the radiation which enters into the first section (14), and of which a second section (16) directly faces the fibre optic (6), has a cross section which is constant in the direction of the middle axis of the light transmission part and has at least one lateral surface (24) which in a largely angularly dependent manner reflects radiation which enters from the first section (14) into the second section (16), **characterized in that**
the fibre optic (6) is an individual rod of a core sheath glass or a fibre bundle of light-conductive fibres (8) which each consist of a core sheath glass,
that at least a part of the lateral surface (22) of the first section (14) is polished and by way of this is designed such that the conditions for a total reflection are fulfilled, and
that at least a part of the second section (16) is formed from a core sheath glass, wherein the core glass (28) and the sheath glass (30) of the second section (16) have a refractive index jump which corresponds to the refractive index jump between the refractive index of the core glass which is used in the fibre optic (6) and the refractive index of the sheath glass which is used in the fibre optic (6).

2. An illumination device according to claim 1, concerning which the light transmission part (10) is arranged distanced to the LED (2) and an intermediate space (20) between the LED (2) and the light transmission part (10) is gas-filled.

3. An illumination device according to one of the preceding claims, concerning which the lateral surface (22) of the first section (14) of the light transmission part (10) is polished and at the outer side is surrounded by a gaseous medium.

4. An illumination device according to one of the claims 1 or 2, concerning which the lateral surface (22) of the first section (14) of the light transmission part (10) is polished, wherein a layer (26) which is highly reflective to the radiation which is emitted by the LED (2) is provided at the outer side of the lateral surface (22).

5. An illumination device according to one of the claims 1 to 4, concerning which an outer lateral surface (34) of the sheath glass is roughened.

6. An illumination device according to one of the claims 1 to 5, concerning which the second section (16) of the light transmission part (10) is formed from a material which is transparent to the radiation emitted by the LED (2) and at its lateral surface is surrounded by a material whose refractive index differs from the refractive index of the material of the second section (16) of the light transmission part (10).

7. An illumination device according to one of the preceding claims, concerning which the second section (16) of the light transmission part (10) is surrounded by a component (36) which is designed for receiving the radiation which is emitted by the LED (2).

8. An illumination device according to one of the preceding claims, concerning which the second section (16) of the light transmission part (10) is formed by a fibre bundle (42), wherein the fibres (44) of the fibre bundle (42) each consist of core-sheath glass.

9. An illumination device according to one of the preceding claims, concerning which the light transmission part (10) is formed from one component.

10. An illumination device according to one of the preceding claims, concerning which the light transmission part (10) is formed by two separate components.

11. An illumination device according to one of the preceding claims, concerning which the cross-sectional profile of the first section (14) of the light transmission part (10) at the end of the first section (14) which faces the LED (2) corresponds to the profile of the light-emitting surface (4) of the LED (2) in the lateral direction.

12. An illumination device according to one of the preceding claims, concerning which the light transmission part (10) is connected to the fibre-optic (6) by way of a temperature-resistant material connection.

## Revendications

1. Appareil d'éclairage avec une LED (2), avec un conducteur optique (6) et un élément de transmission de lumière (10) disposé entre la LED (2) et le conducteur optique (6) et comprenant deux parties (14, 16) dont une première partie (14) est directement orientée vers la LED (2) et présente une section transversale qui croît au fur et à mesure que sa distance de la LED (2) augmente et comprend une surface d'enveloppe (22) qui réfléchit le rayonnement entrant dans la première partie (14) sensiblement indépendamment de l'angle d'incidence, et dont une seconde partie (16) est directement orientée vers le conducteur optique (6), présente une section transversale constante dans la direction de l'axe central de l'élément de transmission de lumière et comprend une surface d'enveloppe (24) qui réfléchit le rayonnement entrant de la première partie (14) dans la seconde partie (16) sensiblement en fonction de l'angle d'incidence, **caractérisé en ce que** le conducteur optique (6) est une tige unique en un verre de noyau-gaine ou un faisceau de fibres de fibres conductrices de lumière (8) qui sont constituées chacune de verre de noyau-gaine,
**en ce qu'**au moins une partie de la surface d'enveloppe (22) de la première partie (14) est polie et est ainsi formée de façon telle que les conditions pour une réflexion totale soient remplies et **en ce qu'**au moins une partie de la seconde partie (16) est formée en un verre de noyau-gaine, le verre de noyau (28) et le verre de gaine (30) de la seconde partie (16) présentant un saut d'indice de réfraction qui est identique au saut d'indice de réfraction entre l'indice de réfraction du verre de noyau utilisé pour le conducteur optique (6) et l'indice de réfraction du verre de gaine utilisé pour le conducteur optique (6).

2. Appareil d'éclairage selon la revendication 1 dans lequel l'élément de transmission de lumière (10) est disposé à une distance de la LED (2) et un espace intermédiaire (20) entre la LED (2) et l'élément de transmission de lumière (10) est rempli de gaz.

3. Appareil d'éclairage selon une des revendications précédentes dans lequel la surface d'enveloppe (22) de la première partie (14) de l'élément de transmission de lumière (10) est polie et est entourée, du côté extérieur, d'un médium gazeux.

4. Appareil d'éclairage selon l'une des revendications 1 ou 2 dans lequel la surface d'enveloppe (22) de la première partie (14) de l'élément de transmission de lumière (10) est polie, du côté extérieur de la surface d'enveloppe (22) une couche (26) étant prévue qui est hautement réfléchissante pour le rayonnement émis par la LED (2).

5. Appareil d'éclairage selon l'une des revendications 1 à 4 dans lequel une surface de gaine extérieure (34) du verre de gaine est rendu rugueuse.

6. Appareil d'éclairage selon l'une des revendications 1 à 5 dans lequel la seconde partie (16) de l'élément de transmission de lumière (10) est formée en un matériau transparent pour le rayonnement émis par la LED (2) et est entourée, à sa surface d'enveloppe, d'un matériau dont l'indice de réfraction diffère de l'indice de réfraction du matériau de la seconde partie (16) de l'élément de transmission de lumière (10).

7. Appareil d'éclairage selon l'une des revendications précédentes dans lequel la seconde partie (16) de l'élément de transmission de lumière (10) est entourée d'un composant (36) qui est formé pour recevoir le rayonnement émis par la LED (2).

8. Appareil d'éclairage selon l'une des revendications précédentes dans lequel la seconde partie (16) de l'élément de transmission de lumière (10) est constituée d'un faisceau de fibres (42), les fibres (44) du faisceau de fibres (42) étant constituées chacune de verre de noyau-gaine.

9. Appareil d'éclairage selon l'une des revendications précédentes dans lequel l'élément de transmission de lumière (10) est constitué d'un composant.

10. Appareil d'éclairage selon l'une des revendications précédentes dans lequel l'élément de transmission de lumière (10) est constitué de deux composants individuels.

11. Appareil d'éclairage selon l'une des revendications précédentes dans lequel le profil de section transversale de la première partie (14) de l'élément de transmission de lumière (10) correspond, à l'extrémité de la première partie (14) orientée vers la LED (2), au profil de la surface émettrice de lumière (4) de la LED (2) en direction latérale.

12. Appareil d'éclairage selon l'une des revendications précédentes dans lequel l'élément de transmission de lumière (10) est relié au conducteur optique (6) au moyen d'une liaison par adhérence de matière.
